# EUROPÄISCHE PATENTSCHRIFT

(11) **EP 2 120 918 B1**
(45) Veröffentlichungstag und Bekanntmachung des Hinweises auf die Patenterteilung: **31.03.2010**
(21) Anmeldenummer: 07846524.2
(22) Anmeldetag: 31.10.2007
(51) Int. Cl.: A61K 31/216, A61K 31/728, A61P 13/00, A61P 13/10

(54) **PHARMAZEUTISCHE ZUSAMMENSETZUNG ZUR BEHANDLUNG VON INKONTINENZ**
PHARMACEUTICAL COMPOSITION FOR TREATING INCONTINENCE
COMPOSITION PHARMACEUTIQUE POUR LE TRAITEMENT DE L'INCONTINENCE

(30) Priorität: 19.01.2007 DE 102007003765
(43) Veröffentlichungstag der Anmeldung: 25.11.2009
(73) Patentinhaber: Farco-Pharma GmbH, 50670 Köln (DE)
(72) Erfinder: VESTWEBER, Anna-Maria, 51399 Burscheid (DE)
(74) Vertreter: Gesthuysen, von Rohr & Eggert
(86) Internationale Anmeldenummer: PCT/EP2007/009487
(87) Internationale Veröffentlichungsnummer: WO 2008/086834

(56) Entgegenhaltungen:
- EP-A- 0 572 932
- WO-A-98/43555
- WO-A-2006/004759
- US-A1- 2006 040 894

## Beschreibung

Die vorliegende Erfindung betrifft eine Zusammensetzung, insbesondere eine pharmazeutische Zusammensetzung, zur Behandlung von Erkrankungen des Urogenitaltraktes. Bei den Erkrankungen handelt es sich bevorzugt um insbesondere im Zusammenhang mit einer Hyperaktivität und/oder Hyperreflexie des Detrusorapparates bzw. des Detrusors (*Musculus detrusor* bzw. *Musculus detrusor vesicae*) stehende Harninkontinenz. Die Zusammensetzung nach der Erfindung enthält in Kombination Oxybutynin bzw. dessen Salze und Hyaluronsäure bzw. dessen Salze.

Des weiteren betrifft die vorliegende Erfindung ein Behältnis und eine Applikationsvorrichtung zur Instillation oder Applikation in den Urogenitaltrakt, welche jeweils die erfindungsgemäße Zusammensetzung enthalten.

Schließlich betrifft die vorliegende Erfindung die Verwendung der Zusammensetzung nach der Erfindung zur prophylaktischen oder therapeutischen Behandlung von Erkrankungen des Urogenitaltraktes, insbesondere von Harninkontinenz, wobei es sich hierbei insbesondere um in Zusammenhang mit einer Hyperaktivität und/oder Hyperreflexie des Detrusorapparates bzw. des Detrusors stehende Harninkontinenz handelt.

Der Aufbau bzw. die Anatomie sowie die Funktion des Urogenitaltraktes, insbesondere der Harnblase, sind gut untersucht. So umfaßt der Blasenkörper glatte Muskulatur bzw. glatte Muskelfasern. Hierzu zählt der sogenannte *Musculus detrusor*, welcher gleichermaßen als Detrusor bezeichnet wird, und ein Bestandteil des sogenannten Detrusorapparates ist bzw. diesen maßgeblich bildet. Beim Vorgang des Harnlassens führt eine Kontraktion des Detrusors neben einer Erhöhung des intravesikalen Drucks zu einer Verkürzung und Erweiterung der proximalen Urethra und somit zu einer Senkung des Abflußwiderstandes. Diese Vorgänge beim Harnlassen werden durch eine vermehrte Parasympatikusaktivität gesteuert. Darüber hinaus besitzt die Blase auch eine sympathische Innervation. Eine α-adrenerge Stimulation führt im Gegenzug zur Zunahme des Abflußwiderstandes, wobei die diesbezüglichen Rezeptoren vorwiegend im Blasenhals und in der proximalen Urethra, dem sogenannten *Sphincter internus* liegen. Eine β-adrenerge Stimulation führt wiederum zu einer Erschlaffung bzw. Relaxation des Blasenkörpers, wobei die diesbezüglichen Rezeptoren vorwiegend in den Blasenkörpern lokalisiert sind.

Insgesamt bilden sympathische und parasympathische Nervenfasern in der Blasenwand ein dichtes Geflecht. Ein willkürlicher Verschluß der Harnröhre ist durch den quergestreiften *Musculus sphincter urethreae*, welcher synonym auch als *Sphincter externus* bzw. *Rabdosphincter* bezeichnet wird, möglich. Der Tonus dieses Muskels nimmt mit zunehmender Blasendehnung kontinuierlich zu, bei der Miktion erschlafft er normalerweise vollständig. Die willkürliche Kontraktion des *Sphincter externus* wird vom *Lobos paracentralis* an der Mantelkante beider Hirnhemisphären gesteuert.

Somit fördert eine Sympathikusaktivierung die Harnspeicherung, eine Parasympathikusaktivierung im Zusammenhang mit der Kontraktion des Detrusors und einer willkürlichen Erschlaffung des *Sphincter externus* dagegen die Blasenentleerung.

Die Harninkontinenz (*Inkontinentia urinae*) stellt ein weit verbreitetes Krankheitsbild dar. Nach verschiedenen Statistiken und Hochrechnungen sind allein in der Bundesrepublik Deutschland 5 bis 8 Millionen Menschen hiervon betroffen, wobei die Tendenz steigend ist. Bei den von Harninkontinenz betroffenen Personen ist der Leidensdruck enorm, insbesondere da mit dieser Erkrankung nicht nur bedeutsame soziale Probleme verbunden sind, sondern auch gravierende hygienische Beeinträchtigungen vorliegen. Die Ursachen für Inkontinenz sind vielfältig, sie können an bzw. in der Harnblase, den hambildenden Organen, den beteiligten Nervensträngen und der Reizverarbeitung im Gehirn liegen.

Neben der sogenannten Streßinkontinenz, welche synonym auch als Belastungsinkontinenz bezeichnet wird, der Überlaufinkontinenz, bei welcher eine ständig übervolle Harnblase infolge von Abflußstörungen vorliegt, und der sogenannten extraurethralen Inkontinenz, bei welcher der Harnabgang aus Öffnungen, die den normalen Wege des Harntraktes umgehen, erfolgt, umfaßt das Krankheitsbild der Harninkontinenz auch die sogenannte Dranginkontinenz (synonym auch als Urge-Kontinenz bezeichnet) und die Reflexinkontinenz.

Bei der Dranginkontinenz, insbesondere der motorischen Dranginkontinenz, liegt eine Hyperaktivität des Detrusors vor. Diese Form der Dysfunktion ist oftmals durch einen unwillkürlichen Harnrhythmus aus der Harnröhre in Verbindung mit einem mitunter starken, nicht unterdrückbaren, imperativen Harndrang gekennzeichnet. Ursache kann eine motorische bzw. neuromuskuläre Überaktivität des Detrusors und somit eine Detrusorhyperaktivität sein.

Die motorische Überaktivität des Detrusors kann zudem begleitet werden von chronischen Blasenauslaßobstruktionen, wie Prostatahyperplasie bzw. Harnröhrenstrikturen, entzündlichen oder neoplastischen intravesikalen Prozessen, degenerativen Veränderungen des Detrusors oder psychovegetativen Störungen. Bei der ideopatischen oder neurogenen Detrusorhyperaktivität stellt sich neben dem zuvor angesprochenen imperativen Harndrang einhergehend mit einer Harninkontinenz oftmals auch eine Pollakisurie ein.

Was den Patomechanismus der motorischen Dranginkontinenz anbelangt, so kann hier ein übermäßiger Zustrom sensibler Afferenzen oder eine unzureichende Hemmung des pontinen oder spinalen Miktionszentrums vorliegen, so daß das physiologische Gleichgewicht zwischen Blasenfüllung und zunehmender Unterdrückung des Miktionsreflexes verloren gehen kann. Dies kann das Vorliegen einer Detrusorhyperaktivität einhergehend mit ungehemmten Detrusorkontraktionen im Hinblick auf eine erhöhte Harninkontinenz verstärken. Denn durch die Hyperaktivität des Detrusors bzw. der ungehemmten bzw. übermäßig starken Kontraktionen des Detrusors wird auch bei geringen Befiillungsgraden der Harnblase ein übermäßiger Druck insbesondere auf den Blasenausgang ausgeübt, so daß schon bei bereits geringfügigen Füllungen der Harnblase ein starker Harndrang auftritt.

Bei der motorischen Dranginkontinenz können zudem die Nerven, die den für die Entleerung der Harnblase zuständigen Detrusor innervieren, enthemmt sein, was zu einer vorzeitigen, manchmal krampfartigen Detrusorkontraktion einhergehend mit starkem Harndrang führt.

Wird die Detrusorhyperaktivität durch eine neurologische Erkrankung verursacht, spricht man im allgemeinen von einer sogenannten Detrusorhyperreflexie, welche oftmals zu einer sogenannten Reflexinkontinenz führen kann. Die Reflexinkontinenz kann durch eine Störung oder Zerstörung der vom Gehirn ausgehenden Hemmungsbahnen hervorgerufen werden und mit einem Überwiegen der Aktivitätsimpulse des Reflexbogens zwischen Harnblase einerseits und Blasenzentrum im Kreuzteil des Rückenmarks andererseits einhergehen. Als Folge können sich auch hier reflexartige Detrusorkontraktionen mit Harnabgang einstellen. Somit kann sich eine spinale Reflexinkontinenz als Folge einer kompletten supranukleären bzw. suprasakralen Läsion des Rükkenmarks entwickeln, durch welche die zentrale Kontrolle des sakralen Miktionszentrums entfällt. Nach dem Abklingen der initialen Schockphase wird der spinale Reflexbogen durch afferente Impulse unterschiedlicher Genese aktiviert und die Blase durch reflektorische Detrusorkontraktionen entleert, ohne daß der Patient einen Harndrang verspürt oder den Urinabgang bemerkt.

Bei einer supranukleären Reflexblase tritt zugleich meist eine Detrusor/*Sphincter externus*-Dyssynergie auf. Während der Detrusorkontraktion kontrahiert sich gleichzeitig der Schließmuskel, so daß der Detrusormuskel ständig gegen einen hohen Auslaßwiderstand ankämpfen muß. Dieser Umstand bewirkt über eine Muskelhypertrophie des Detrusors einen Umbau der Blasenwand mit Verlust der Blasenelastizität. Dadurch entstehen hohe intravesikale Speicherdrücke, die letztlich über eine progrediente Abflußstörung der oberen Harnwege zur Niereninsuffizienz führen können.

Die Ursache für eine Detrusorhyperreflexie kann in neurologischen Erkrankungen liegen, beispielsweise bei Multipler Sklerose, Tumoren des Rückenmarks, Bandscheibenprolaps, Querschnittslähmung durch Verletzung oder zerebrovaskulären Erkrankungen.

Bei der Harninkontinenz bzw. bei Blasenstörungen ist oftmals eine einhergehende entzündliche Veränderung des Urothels zu beobachten, was dadurch bedingt sein kann, daß die Glykosaminoglykanschicht in der Harnblase abgebaut wird bzw. degeneriert ist. Dies ist auf eine Störung der Zusammensetzung der Glykosaminoglykanschicht der Blasenschleimhaut, welche Hyaluronsäure und Chondroitinsulfat enthält, zurückzuführen. Hieraus folgt oftmals eine den Patienten zusätzlich belastende Schmerzsymptomatik, die den Leidensdruck weiter verstärkt. Zudem können aufgrund der geschädigten Glykosaminoglykanschicht Bakterien, Mikrokristalle, Proteine und/oder gelöste Bestandteile des Harns direkt in tiefere Schichten der Blasenschleimhaut eindringen und zu einer weiteren Schädigung einhergehend mit Schmerzen führen. Dabei kann die mit der Harninkontinenz auftretende Schädigung der Glykosaminoglykanschicht auch im Zusammenhang mit einer interstitiellen Cystitis stehen.

Vor diesem Hintergrund besteht aufgrund der Schwere und der Verbreitung der Harninkontinenz ein großer Bedarf, geeignete pharmazeutische Zusammensetzungen und Therapieformen bereitzustellen.

Was die im Stand der Technik vorgesehene Medikation bzw. Therapie der zur beschriebenen Formen der Harninkontinenz anbelangt, so ist insbesondere in bezug auf die motorische Dranginkontinenz als auch auf die Reflexinkontinenz oftmals eine Behandlung mit Substanzen vorgesehen, welche anticholinerge Eigenschaften aufweisen. Hierbei handelt es sich beispielsweise um die Substanzen Scopolamin, Trospiumchlorid sowie Emepronium. Derartige Substanzen werden im Stand der Technik oftmals systemisch verabreicht, wobei insbesondere eine Verabreichung in Form von peroral aufzunehmenden Tabletten vorgesehen ist. Nachteilig hierbei ist jedoch, daß die Wirksubstanz über den Gastrointestinaltrakt aufgenommen wird und somit erst auf indirektem Wege zum Wirkort gelangt. Zudem ist aufgrund der systemischen Verabreichung bzw. Applikation eine erhöhte Dosis bzw. Menge an Wirksubstanz erforderlich. In diesem Zusammenhang ist in bezug auf die orale bzw. perorale Applikation der vorgenannten Substanzen nachteilig, daß oftmals die für Anticholinergika typischen Nebenwirkungen auftreten, was die Akzeptanz dieser Therapieform beim Patienten verringert. Als typische, mit der peroralen Verabreichung in Verbindung stehende Nebenwirkungen können Mundtrokkenheit, Akkomodationsstörungen, Hydriasis, Augeninnendruckerhöhung, Übelkeit, Obstipation sowie Tachykardie aufgezählt werden. Dabei können die Nebenwirkungen derart stark sein, daß die Verabreichung ohne Therapieerfolg abgebrochen werden muß.

WO 9843555 und EP572932 offenbaren die Verwendung von Oxybutynin gegen Inkontinenz, und Hyaluronsaure als Hilfstoff.

Als weitere therapeutische Maßnamen kommt die Verabreichung von Flavoxat, was direkt relaxierend auf die glatte Muskulatur wirkt, Calciumantagonisten, Nifedipin, Verapamil und Imipramin in Frage. Die vorgenannten Wirksubstanzen sind jedoch hinsichtlich des Therapieerfolges nicht immer optimal. Zudem führt Imipramin mitunter zu einer Erhöhung des Tonus des *Sphincter internus*, was unerwünscht ist, insbesondere wenn dessen Tonus bei der zuvor beschriebenen Detrusor/*Sphincter externus*-Dyssynergie ohnehin gesteigert ist. Zudem sind zahlreiche Gegenanzeigen, Anwendungsbeschränkungen und Nebenwirkungen zu berücksichtigen, welche denen der tri- und tetracyclischen Antidepressiva entsprechen.

Insgesamt führen somit die im Stand der Technik vorgesehenen medikamentösen Ansätze zur Behandlung der Harninkontinenz, insbesondere der mit der Hyperaktivität bzw. Hyperreflexie des Detrusors in Zusammenhang stehenden Harninkontinenz, wie insbesondere der motorischen Dranginkontinenz und Reflexinkontinenz, nicht immer zu einem ausreichenden Therapieerfolg bzw. zu mitunter erheblichen Nebenwirkungen.

Somit liegt der vorliegenden Erfindung das Problem zugrunde, eine Zusammensetzung, insbesondere pharmazeutische Zusammensetzung, bereitzustellen, welche sich zur Behandlungen von Erkrankungen des Urogenitaltraktes, vorzugsweise von Harninkontinenz eignet, und welche die zuvor geschilderten Nachteile des Standes der Technik zumindest weitgehend vermeidet oder zumindest abschwächt. Dabei soll insbesondere eine verbesserte Wirksamkeit bei gleichzeitig verringerten Nebenwirkungen gewährleistet sein.

Gegenstand der vorliegenden Erfindung - gemäß einem **ersten** Aspekt - ist somit eine Zusammensetzung, insbesondere pharmazeutische Zusammensetzung, welche sich zur Behandlung von Erkrankungen des Urogenitaltraktes, insbesondere von Harninkontinenz, eignet und - in Kombination und jeweils in wirksamen, insbesondere pharmazeutisch wirksamen Mengen -
- Oxybutynin und/oder dessen Salze sowie
- Hyaluronsäure und/oder deren Salze
enthält.

Der Begriff "pharmazeutische Zusammensetzung" bzw. "pharmazeutische Zubereitung" oder dergleichen, wie er im Rahmen der vorliegenden Erfindung verwendet wird, ist sehr umfassend zu verstehen und bezeichnet nicht nur pharmazeutische Präparate bzw. Pharmazeutika als solche, sondern auch sogenannte Medizinprodukte, homöopathische Mittel oder dergleichen.

Bei allen nachstehend genannten relativen bzw. prozentualen gewichtsbezogenen Mengenangaben ist zu beachten, daß diese im Rahmen der erfindungsgemäßen Zusammensetzung bzw. Kombination vom Fachmann derart auszuwählen sind, daß sie sich in der Summe unter Einbezug der Komponenten bzw. Inhaltsstoffe bzw. Bestandteile, insbesondere wie nachfolgend definiert, stets um 100 Gew.-% ergänzen. Dies versteht sich für den Fachmann aber von selbst.

Die vorliegende Erfindung zeichnet sich durch die Verwendung von zwei Wirksubstanzen - Oxybutynin und/oder dessen Salzen einerseits und Hyaluronsäure und/oder deren Salze andererseits - im Rahmen der erfindungsgemäßen Zusammensetzung aus. Denn die Anmelderin hat in völlig überraschender Weise herausgefunden, daß durch die spezielle Kombination der vorgenannten Substanzen eine besonders wirksame Zusammensetzung nach der Erfindung bereitgestellt wird, welche sich in hohem Maße zur Behandlung von Erkrankungen des Urogenitaltraktes, vorzugsweise von Harninkontinenz eignet. In diesem Zusammenhang handelt es sich bei der zu behandelnden Harninkontinenz vorzugsweise um eine im Zusammenhang mit Fehlfunktionen des Detrusors (*Musculus detrusor* bzw. *Musculus detrusor vesicae*) stehende Harninkontinenz, wie die zuvor beschriebene Dranginkontinenz, insbesondere motorische Dranginkontinenz, sowie die zuvor beschriebene Reflexinkontinenz.

Die vorliegende Erfindung zielt somit insbesondere auf solche Harninkontinenzerkrankungen ab, die mit einer Hyperaktivität oder Hyperreflexie des Detrusors in Zusammenhang stehen, wie es bei der Dranginkontinenz, insbesondere der motorischen Dranginkontinenz, und der Reflexinkontinenz der Fall ist. Darüber hinaus eignet sich die erfindungsgemäße Zusammensetzung - wie nachfolgend noch ausführlich geschildert - auch zur Behandlung von entzündlichen Erkrankungen des Urogenitaltraktes, wie beispielsweise der interstitiellen Cystitis.

Dabei ist es im Rahmen der vorliegenden Erfindung in eindrucksvoller Weise gelungen, eine hochwirksame Zusammensetzung mit gleichzeitig signifikant verringerten Nebenwirkungen bereitzustellen.

Bei der im Rahmen der vorliegenden Erfindung verwendeten Wirksubstanz Oxybutynin handelt es sich um den internationalen Freinamen für das spasmolytisch wirkende Anticholinergikum (±)-α-Cyclohexyl-α-hydroxyphenylessigsäure-4-(diethylamino)-2-butinylester gemäß der folgenden Strukturformel:

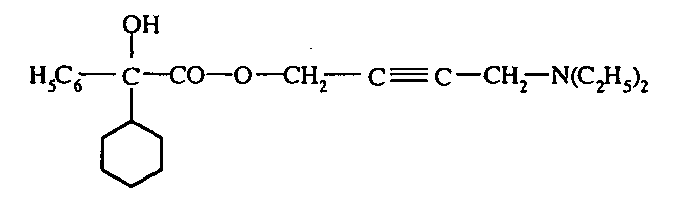

Die Wirkeigenschaften von Oxybutynin sind dahingehend zu beschreiben, daß es an der glatten Muskulatur - und somit auch an dem zuvor beschriebenen Detrusormuskel der Harnblase - relaxierend wirkt und somit einen spasmolytischen Effekt aufweist. Darüber hinaus hemmt Oxybutynin die Wirkung des Neurotransmitters Acetylcholin an der glatten Muskulatur, was einem anticholinergen Effekt entspricht. Darüber hinaus wird Oxybutynin eine analgetische und eine lokalanästhetische Wirkung zugesprochen. Genauer gesagt kann - ohne sich auf diese Theorie beschränken zu wollen - Oxybutynin an parasympathischen muskarinergen Rezeptoren kompetetiv binden, es fungiert somit als Acetylcholinantagonist. Aufgrund dieser Eigenschaften kann - ohne sich auf diese Theorie beschränken zu wollen - aufgrund der anticholinergen Wirksamkeit von Oxybutynin, insbesondere in Zusammenhang mit den spasmolytischen Eigenschaften, eine relaxierende Wirkung auf den Detrusor der Harnblase ausgeübt werden, was die unerwünschten Spontankontraktionen des Detrusors bzw. den erhöhten Detrusortonus reduziert. Es resultiert folglich eine Verringerung des maximalen Detrusordruckes während des Füllens der Harnblase mit Harn, so daß das Blasenvolumen und die Blasenkapazität erhöht wird und somit insgesamt der Harndrang verringert wird. Dabei ist es im Rahmen der vorliegenden Erfindung in völlig überraschender Weise gelungen, die Wirksamkeit von Oxybutynin durch die gezielte Kombination mit Hyaluronsäure bzw. deren Salze zu steigern - wie nachfolgend noch ausführlich erörtert.

Die erste Komponente der erfindungsgemäßen Zusammensetzung - das Oxybutynin und/oder dessen Salze - kann vorzugsweise auf einer sterilen oder hochgereinigten Lösung oder Suspension des Hydrochlorids von Oxybutynin basieren.

Für weitergehende Einzelheiten zu Oxybutynin bzw. dessen Salze kann auf Römpp Chemielexikon, 10. Auflage, Band 4, 1998, Georg Thieme Verlag Stuttgart/New York, Seite 3081, Stichwort: "Oxybutynin" sowie die dort referierte Literatur verwiesen werden, wobei der gesamte Offenbarungsgehalt der vorgenannten Literatur hiermit durch Bezugnahme eingeschlossen ist.

Der volumenbezogene Wirkstoffgehalt an Oxybutynin bzw. dessen Salze beträgt 0,01 bis 0,2 mg/ml, insbesondere 0,01 bis 0,15 mg/ml, vorzugsweise 0,02 bis 0,1 mg/ml, bevorzugt 0,03 bis 0,08 mg/ml, besonders bevorzugt 0,04 bis 0,07 mg/ml, wobei sich die vorgenannten Wirkstoffgehalte auf das Volumen der Zusammensetzung und berechnet als Oxybutynin beziehen. Folglich sind die vorgenannten Wirkstoffgehalte in bezug auf die erfindungsgemäß verwendbaren Salze von Oxybutynin, vorzugsweise Oxybutyninhydrochlorid, unter Einbezug der entsprechenden Molmassen anzugleichen. Gemäß einer erfindungsgemäß ganz besonders bevorzugten Ausführungsform enthält die erfindungsgemäße Zusammensetzung somit etwa 0,0567 mg/ml Oxybutynin bzw. 0,0625 mg/ml Oxybutyninhydrochlorid. Bei einem erfindungsgemäß bevorzugt applizierten Volumen der erfindungsgemäßen Zusammensetzung von 50 ml enthält die erfindungsgemäße Zusammensetzung in diesem Volumen somit 0,5 bis 10 mg, insbesondere 0,5 bis 7,5 mg, vorzugsweise 1 bis 5 mg, bevorzugt 1,5 bis 4 mg und besonders bevorzugt 2 bis 3,5 mg Oxybutynin. Gemäß einer erfindungsgemäß ganz besonders bevorzugten Ausführungsform enthält die Zusammensetzung nach der Erfindung somit etwa 2,84 mg Oxybutynin bzw. 3,125 mg Oxybutyninhydrochlorid in einem Volumen von 50 ml..

Was die erfindungsgemäße Zusammensetzung weiterhin anbelangt, so kann diese das Oxybutynin bzw. dessen Salze in Mengen von 0,001 bis 0,02 Gew.-%, insbesondere 0,001 bis 0,015 Gew.-%, vorzugsweise 0,002 bis 0,01 Gew.-%, bevorzugt 0,003 bis 0,008 Gew.-%, besonders bevorzugt 0,004 bis 0,007 Gew.-%, enthalten, bezogen auf das Gesamtgewicht der Zusammensetzung und berechnet als Oxybutynin.

Im Rahmen der vorliegenden Erfindung wird das Oxybutynin vorzugsweise in Form eines insbesondere sauren Salzes, vorzugsweise in Form eines sauren Additionssalzes, bevorzugt in Form eines Hydrochlorids, eingesetzt. Demnach wird erfindungsgemäß besonders bevorzugt Oxybutyninhydrochlorid verwendet, welches im Handel als solches beispielsweise von der Firma Sigma-Aldrich, St. Louis, Vereinigte Staaten von Amerika, erhältlich ist. Oxybutyninhydrochlorid zeichnet sich dadurch aus, daß es bei gleichzeitig guter Wirksamkeit und Verträglichkeit eine erhöhte Löslichkeit in Wasser aufweist.

Was die weitere Komponente der erfindungsgemäßen Zusammensetzung - nämlich die Hyaluronsäure bzw. deren Salze - anbelangt, so kann diese in erfindungsgemäß bevorzugter Weise eine relativ geringe mittlere Molmasse aufweisen. Denn was die mittlere Molmasse - im Rahmen der vorliegenden Erfindung synonym auch als Molekulargewicht bezeichnet - der erfindungsgemäß verwendeten Hyaluronsäure bzw. deren Salze betrifft, so kann diese in einem Bereich von 50 bis 195 kDa (Kilodalton), insbesondere 100 bis 195 kDa, vorzugsweise 150 bis 190 kDa, bevorzugt 175 bis 190 kDa liegen. So kann erfindungsgemäß beispielsweise eine Hyaluronsäure bzw. deren Salze mit einem Molekulargewicht von etwa 180 kDa verwendet werden.

Denn die Anmelderin hat in völlig überraschender Weise herausgefunden, daß durch den Einssatz einer speziellen Hyaluronsäure der vorgenannten Art bzw. von deren Salze hinsichtlich der Behandlung von Harninkontinenz besonders gute Ergebnisse erzielt werden. Diesbezüglich kommt- wie die Anmelderin überraschend herausgefunden hat - auch dem Molekulargewicht der Hyaluronsäure eine entscheidende Rolle zu. Ohne sich auf eine Theorie festlegen zu wollen, kann dies damit begründet werden, daß Hyaluronsäure bzw. deren Salze mit geringerem Molekulargewicht gewissermaßen besser in die geschädigten Urothel- bzw. Zellschichtstrukturen der Harnblase eindringen bzw. besser mit diesen interagieren und somit sozusagen die Harnblaseninnenwandung besser "auskleiden" bzw. abdecken kann. Gleichzeitig führt die Verwendung einer Hyaluronsäure bzw. deren Salze mit relativ geringem Molekulargewicht dazu, daß gewissermaßen - in Abstimmung mit der weiteren Wirksubstanz Oxybutynin bzw. dessen Salze - eine besonders effektive Matrix bereitgestellt wird, in die - ohne sich auf diese Theorie beschränken zu wollen - das Oxybutynin sozusagen einlagert bzw. integriert wird. Dies führt dazu, daß das Oxybutynin über einen längeren Zeitraum im Rahmen eines Controlled-Release-Prozesses am Wirkort verfügbar ist. Hieraus resultiert gleichermaßen eine bessere Bioverfügbarkeit, so daß auch die Menge an Wirksubstanz in bezug auf Oxybutynin im Rahmen der erfindungsgemäßen Zusammensetzung herabgesetzt werden kann. Es resultiert somit im Rahmen der erfindungsgemäßen Kombination eine hohe Wirksamkeit bei gleichzeitiger Verringerung der zuvor genannten, in Verbindung mit Anticholinergika auftretenden Nebenwirkungen.

Der volumenbezogene Wirkstoffgehalt von Hyaluronsäure bzw. deren Salzen beträgt in der erfindungsgemäßen Zusammensetzung 0,2 bis 2,0 mg/ml, insbesondere 0,3 bis 1,6 mg/ml, vorzugsweise 0,4 bis 1,2 mg/ml, bevorzugt 0,5 bis 1,0 mg/ml, besonders bevorzugt 0,7 bis 0,9 mg/ml, bezogen auf das Volumen der vorzugsweise in flüssiger Form vorliegenden Zusammensetzung nach der Erfindung. Dies bedeutet, daß bei einem erfindungsgemäß bevorzugt applizierten bzw. instillierten Volumen der erfindungsgemäßen Zusammensetzung von 50 ml folglich 10 bis 100 mg, insbesondere 15 bis 80 mg, bevorzugt 20 bis 60 mg, besonders bevorzugt 25 bis 50 mg, besonders bevorzugt 35 bis 45 mg Hyaluronsäure und/oder deren Salze in der Zusammensetzung nach der Erfindung in dem Volumen von 50 ml enthalten sind. Gemäß einer erfindungsgemäß ganz besonders bevorzugten Ausführungsform enthält die Zusammensetzung nach der Erfindung in einem Volumen von 50 ml etwa 40 mg Hyaluronsäure bzw. deren Salze.

Was die erfindungsgemäße Zusammensetzung weiterhin anbelangt, so kann diese die Hyaluronsäure bzw. deren Salze in relativen, gewichtsbezogenen Mengen von 0,02 bis 0,2 Gew.-%, insbesondere 0,03 bis 0,16 Gew.-%, vorzugsweise 0,04 bis 0,12 Gew.-%, bevorzugt 0,05 bis 0,01 Gew.-%, bezogen auf das Gesamtgewicht der Zusammensetzung und berechnet als Hyaluronsäure, enthalten. Dennoch kann es einzelfallbedingt oder anwendungsbezogen erforderlich sein, von den vorgenannten Mengen abzuweichen, ohne daß der Rahmen der vorliegenden Erfindung verlassen ist. Somit kann die Menge an Hyaluronsäure bzw. deren Salzen in weiten Bereichen variieren.

Die erfindungsgemäße Zusammensetzung zeichnet sich dadurch aus, daß die verwendete Hyaluronsäure bzw. deren Salze bevorzugt nichttierischen Ursprungs ist. So kann die Hyaluronsäure bzw. deren Salze beispielsweise bakteriellen Ursprungs sein. Diesbezüglich kann die Hyaluronsäure und/oder deren Salze fermentativ von Bakterien der Gattung *Streptococcus*, insbesondere *Streptococcus lancefields*, bevorzugt *Streptococcus lancefields* Stamm A, gewonnen sein. Die diesbezügliche Isolierung der Wirksubstanzen ist dem Fachmann als solche geläufig. Weiterhin kann die erfindungsgemäß verwendete Hyaluronsäure aber auf pflanzlichen Ursprungs sein.

Die Anmelderin hat nicht zuletzt aufgrund ihrer intensiven Forschungstätigkeit in überraschender Weise herausgefunden, daß die Verwendung einer nichttierischen Hyaluronsäure die pharmazeutische Wirksamkeit der erfindungsgemäßen Zusammensetzung in bezug auf die Behandlung von Harninkontinenz signifikant verbessert. Bei der nichttierischen, insbesondere bakteriell gewonnen Hyaluronsäure handelt es sich um ein besonders reines Produkt mit definierten chemischen und physikalischen Eigenschaften, welches hochwirksam ist. Zudem ist die nichttierische Hyaluronsäure gut verträglich, da eine Kontamination bzw. Verunreinigung mit anderen Stoffen, wie es oftmals bei tierisch gewonnenen Produkten der Fall ist, nicht vorliegt. In diesem Zusammenhang ist die erfindungsgemäße Hyaluronsäure beispielsweise zumindest im wesentlichen frei von Endotoxinen, die bei der Anwendung zu Unverträglichkeitsreaktionen führen können. Der Endotoxingehalt sollte in bezug auf die erfindungsgemäß verwendete Hyaluronsäure weniger als 1 ppm in der fertigen Lösung betragen, so daß auf diese Weise wirksam Allergien bzw. allergische Reaktionen bei der Applikation bzw. Instillation vermieden werden. Eine weitere Erhöhung der Reinheit kann in bezug auf die Hyaluronsäure im Rahmen der vorliegenden Erfindung beispielsweise durch insbesondere mehrfaches Waschen der Hyaluronsäure bzw. deren Salze in einer Na-OH-Lösung erreicht werden. Somit weist Hyaluronsäure nichttierischen Ursprungs eine besonders hohe Reinheit und Homogenität auf. Erfindungsgemäß kann gleichermaßen eine Hyaluronsäure pflanzlichen Ursprungs verwendet werden.

Gemäß einer erfindungsgemäß bevorzugten Ausführungsform wird die Hyaluronsäure bzw. deren Salze als Alkalihyaluronat eingesetzt, wobei es sich diesbezüglich vorzugsweise um Natriumhyaluronat handelt.

Die zweite Komponente der erfindungsgemäßen Zusammensetzung - die Hyaluronsäure und/oder deren Salze - kann vorzugsweise auf einer sterilen und hochgereinigten Lösung oder Suspension des Natriumsalzes der Hyaluronsäure basieren.

Für weitergehende Einzelheiten zum Begriff der Hyaluronsäure bzw. deren Salze kann auf Römpp Chemielexikon, 10. Auflage, Band 3, 1997, Georg Thieme Verlag Stuttgart/New York, Seite 1820, Stichwort: "Hyaluronsäure", sowie die dort referierte Literatur verwiesen werden, wobei der gesamte Offenbarungsgehalt dieser Literaturstelle hiermit durch Bezugnahme eingeschlossen ist.

Im Rahmen der vorliegenden Erfindung ist es gelungen, die Wirksamkeit der erfindungsgemäßen Zusammensetzung in bezug auf die Behandlung von Harninkontinenz, insbesondere der vorgenannten Art, durch eine gezielte Abstimmung des Mengenverhältnisses zwischen Oxybutynin bzw. dessen Salzen einerseits und Hyaluronsäure bzw. deren Salzen andererseits weiter zu erhöhen. So kann es erfindungsgemäß vorgesehen sein, daß das Mengenverhältnis von Oxybutynin bzw. dessen Salzen einerseits und der Hyaluronsäure bzw. deren Salze andererseits in der Zusammensetzung im Bereich von 1 : 1 bis 1 : 200, insbesondere 1 : 2 bis 1 : 160, vorzugsweise 1 : 4 bis 1 : 60, bevorzugt 1 : 6 bis 1 : 35, besonders bevorzugt 1 : 10 bis 1 : 20, ganz besonders bevorzugt etwa 1 : 14, liegt, berechnet als Oxybutynin zu Hyaluronsäure. Denn die gezielte Abstimmung der Wirksubstanzen führt zu einer besonders guten Wirksamkeit hinsichtlich der Behandlung der vorgenannten Harninkontinenzerkrankungen, da - ohne sich auf eine Theorie festlegen zu wollen - insbesondere bei den vorgenannten Mengenverhältnissen zum einen eine besonders gute Integration der Hyaluronsäure in die mitunter geschädigte Urothelschicht vorliegt und zum anderen eine gewissermaßen optimierte, durch die Hyaluronsäure gebildete Matrixstruktur geschaffen wird, in welche das Oxybutynin sozusagen optimal eingelagert werden kann.

Aufgrund der Verwendung der beiden Wirksubstanzen Oxybutynin und Hyaluronsäure in der erfindungsgemäßen Wirksubstanz wird somit einerseits eine optimale Regeneration des Blasenurothels hervorgerufen, so daß die Permeabilität des Urothels in signifikanter Weise erniedrigt wird, was zu einer deutlichen Linderung der einhergehenden Schmerzsymptome verbunden ist, insbesondere da Reizstoffe nicht mehr so tief in das Urothel bzw. darunterliegende Schichten eindringen können. Zudem liegt eine optimierte Langzeitwirkung des Oxybutynins vor, da dieses gewissermaßen in einer Matrix eingelagert ist was aufgrund der zuvor beschriebenen spasmolytischen und anticholinergen Eigenschaften zu einer langanhaltenden Relaxation insbesondere des Detrusors führt verbunden mit einer Verringerung des Harndrangs.

In Ergänzung kann die erfindungsgemäße Zusammensetzung weiterhin mindestens einen Elektrolyten enthalten, welcher insbesondere in Mengen von 0,005 bis 1,5 Gew.-%, insbesondere 0,05 bis 1,25 Gew.-%, vorzugsweise 0,25 bis 1 Gew.-%, bevorzugt 0,5 bis 0,75 Gew.-% vorliegt, bezogen auf das Gesamtgewicht der Zusammensetzung. Bei dem erfindungsgemäß fakultativ vorgesehenen Elektrolyten kann es sich um einen Elektrolyten handeln, welcher aus organischen oder anorganischen Alkali- oder Erdalkalisalzen sowie deren Mischungen ausgewählt ist. Vorzugsweise handelt es sich bei dem Elektrolyten um ein Alkali- oder Erdalkalisalz. Gemäß einer erfindungsgemäß besonders bevorzugten Ausführungsform handelt es sich bei dem Alkali- bzw. Erdalkalisalz um ein Chlorid und vorzugsweise um Natriumchlorid. Diesbezüglich ist es erfindungsgemäß besonders bevorzugt, wenn der Elektrolyt, vorzugsweise Natriumchlorid, in der vorzugsweise flüssigen erfindungsgemäßen Zusammensetzung in einer Menge von etwa 345 mg bei einem Volumen von 50 ml vorhanden ist. Einzelfallbedingt oder anwendungsbezogen kann es dennoch erforderlich sein, von den vorgenannten Mengen abzuweichen, ohne daß der Rahmen der vorliegenden Erfindung verlassen ist.

Die erfindungsgemäße Zusammensetzung ist nicht auf die vorgenannten Wirkstoffe bzw. Substanzen beschränkt. So kann es erfindungsgemäß vorgesehen sein, daß die Zusammensetzung nach der Erfindung weitere Substanzen enthält:

Gemäß einer erfindungsgemäß bevorzugten Ausführungsform kann die Zusammensetzung nach der Erfindung zudem Chondroitinsulfat, insbesondere in Mengen von 0,02 bis 0,2 Gew.-%, vorzugsweise 0,03 bis 0,16 Gew.-%, bevorzugt 0,04 bis 0,12 Gew.-%, besonders bevorzugt 0,05 bis 0,10 Gew.-% enthalten, bezogen auf das Gesamtgewicht der Zusammensetzung.

In bezug auf das Volumen der Zusammensetzung kann das Chondroitinsulfat in Mengen von 0,2 bis 2,0 mg/ml, insbesondere 0,3 bis 1,6 mg/ml, vorzugsweise 0,4 bis 1,2 mg/ml, bevorzugt 0,5 bis 1,0 mg/ml, besonders bevorzugt 0,7 bis 0,9 mg/ml in der erfindungsgemäßen Zusammensetzung vorliegen.

Was das Chondroitinsulfat anbelangt, so kann es sich hierbei gemäß einer erfindungsgemäß besonders bevorzugten Ausführungsform um Chondroitinsulfat marinen Ursprungs handeln. Erfindungsgemäß bevorzugt wird Chondroitin-4-sulfat (Chondroitinsulfat A), Chondroitin-6-sulfat (Chondroitinsulfat C) und/oder Dermatansulfat (Beta-Heparin bzw. Chondroitinsulfat B), vorzugsweise Chondroitin-4-sulfat und/oder Chondroitin-6-sulfat bzw. deren Mischungen, eingesetzt.

Für weitergehende Einzelheiten zum Begriff der Chondroitinsulfate kann auf Römpp Chemielexikon, 10. Auflage, Band 1, 1996, Georg Thieme Verlag Stuttgart/New York, Seite 736, Stichwort: "Chondroitinsulfate", sowie die dort referierte Literatur verwiesen werden, wobei der gesamte Offenbarungsgehalt der vorgenannten Literatur hiermit durch Bezugnahme eingeschlossen ist.

Die Anmelderin hat völlig überraschend herausgefunden, daß die Zugabe eines Chondroitinsulfats der vorgenannten Art zu der erfindungsgemäßen Zusammensetzung zu einer weiteren Verbesserung der Wirksamkeit der erfindungsgemäßen Zusammensetzung in bezug auf Harninkontinenz führt. Ohne sich diesbezüglich auf eine Theorie festlegen zu wollen, eignen sich Chondroitinsulfate - welche ein Bestandteil der Glykosaminoglykanschicht des Urothels darstellen - in besonderer Weise, um die Glykosaminoglykanschicht insbesondere in Verbindung mit Hyaluronsäure zu regenerieren, so daß dessen Schutzfunktion verbessert ist.

Die erfindungsgemäße Zusammensetzung kann zudem beispielsweise außerdem Dexpanthenol bzw. dessen Derivate, insbesondere Ester, enthalten. Die diesbezüglichen Mengen können im Bereich von 0,0001 bis 5 Gew.-%, vorzugsweise 0,001 bis 4 Gew.-%, bevorzugt 0,01 bis 3 Gew.-%, besonders bevorzugt 0,1 bis 2 Gew.-%, liegen, bezogen auf das Gesamtgewicht der Zusammensetzung. Bei Dexpanthenol handelt es sich um den internationalen Freinamen für D-(+)-2,4-Dihydroxy-N-(3-hydroxypropyl)-3,3-dimethylbutyramid. Hierbei handelt es sich um eine wundheilend wirkende Substanz, welche die Wirkung der erfindungsgemäßen Zusammensetzung unterstützen bzw. steigern kann, beispielsweise durch ein verbessertes Ausheilen von Läsionen des Urothels.

Weiterhin kann die erfindungsgemäße Zusammensetzung außerdem zusätzlich Ectoin bzw. Ectoinderivate, wie Hydroxyectoin, enthalten. Die diesbezüglichen Mengen können im Bereich von 0,0001 bis 5 Gew.-%, vorzugsweise 0,001 bis 4 Gew.-%, bevorzugt 0,1 bis 3 Gew.-%, besonders bevorzugt 0,1 bis 2 Gew.-%, variieren, bezogen auf das Gesamtgewicht der Zusammensetzung. Bei Ectoin bzw. Ectoinderivaten, wie Hydroxyectoin, handelt es sich um Wirksubstanzen zur Behandlung von Hauterkrankungen. Derartige Substanzen können die Wirksamkeit der erfindungsgemäßen Zusammensetzung weiter unterstützen, insbesondere hinsichtlich ihrer positiven Wirkung in bezug auf das Urothel. Für weitere diesbezügliche Ausführungen in bezug auf Ectoin kann verwiesen werden auf die europäische Patentschrift EP 0 887 418 B1, deren gesamter diesbezüglicher Offenbarungsgehalt hiermit durch Bezugnahme eingeschlossen ist.

Weiterhin kann es vorgesehen sein, der erfindungsgemäßen Zusammensetzung - als alleinige zusätzliche Wirksubstanz oder in Ergänzung zu den vorgenannten Wirkstoffen - außerdem mindestens ein Lokalanästhetikum zuzugeben. Der Fachmann ist jederzeit in der Lage, Lokalanästhetika auszuwählen, die in bezug auf die vorliegende Erfindung besonders geeignet sind. Die diesbezüglichen Mengen des Lokalanästhetikums können erfindungsgemäß im Bereich von 0,0001 bis 5 Gew.-%, vorzugsweise 0,001 bis 4 Gew.-%, bevorzugt 0,01 bis 3 Gew.-%, besonders bevorzugt 0,1 bis 2 Gew.-%, liegen, bezogen auf das Gesamtgewicht der Zusammensetzung. Die Zugabe eines Lokalanästhetikums kann die Wirkung der erfindungsgemäßen Zusammensetzung dahingehend unterstützen bzw. verbessern, daß im Zusammenhang mit der Instillation bzw. Applikation der erfindungsgemäßen Zusammensetzung auch eine gewisse Schmerzstillung eintritt. Dies ist besonders vorteilhaft und führt zu einem erhöhten Wohlbefinden von mit der erfindungsgemäßen Zusammensetzung behandelten Patienten.

Schließlich kann die erfindungsgemäße Zusammensetzung außerdem auch Mineralstoffe und/oder Vitamine enthalten. Insbesondere können Zinkverbindungen, vorzugsweise organische Zinkverbindungen, bevorzugt Zinkgluconat, eingesetzt werden. Die diesbezüglichen Mengen können im Bereich von 0,0001 bis 5 Gew.-%, vorzugsweise 0,001 bis 4 Gew.-%, bevorzugt 0,01 bis 3 Gew.-%, besonders bevorzugt 0,1 bis 2 Gew.-%, liegen, bezogen auf das Gesamtgewicht der Zusammensetzung. Zinkverbindungen der vorgenannten Art weisen eine entzündungshemmende Wirkung auf.

Neben den vorgenannten Wirk- und/oder Inhaltsstoffen kann die erfindungsgemäße Zusammensetzung außerdem übliche pharmazeutische Zusatz-und/oder Hilfsstoffe enthalten. Diese können insbesondere ausgewählt sein aus der Gruppe von Stabilisierungs-, Färbe-, Puffer-, Riech-, Streck-, Binde-, Netz- und/oder Konservierungsstoffen und deren Kombinationen. Der Fachmann ist jederzeit in der Lage, die diesbezüglich zu wählenden Mengen in bezug auf die erfindungsgemäße Zusammensetzung anzupassen.

Die erfindungsgemäße Zusammensetzung liegt vorzugsweise in flüssiger Form vor, beispielsweise in Form einer wäßrigen Lösung bzw. in Form einer vorzugsweise wäßrigen Suspension, wobei Wasser als Lösemittel erfindungsgemäß bevorzugt ist. Aufgrund des Vorliegens der Zusammensetzung in flüssiger Form eignet sich die erfindungsgemäße Zusammensetzung zur vorzugsweise topischen Applikation bzw. Instillation insbesondere mittels hierfür geeigneter Applikations- bzw. Instillationsvorrichtungen in den Urogenitalbereich, insbesondere in die Harnblase. Dabei ist es selbstverständlich, daß die erfindungsgemäße Zusammensetzung steril ist, um während der Anwendung bakterielle Infektionen zu vermeiden.

Was den pH-Wert der erfindungsgemäßen Zusammensetzung anbelangt, so kann dieser im Bereich vom 5,5 bis 7,5, insbesondere 6,0 bis 7,0, vorzugsweise 6,2 bis 6,8, liegen. Erfindungsgemäß ist es somit bevorzugt, daß der pH-Wert in einem leicht sauren Bereich liegt, da dies die Löslichkeit von Oxybutynin bzw. dessen Salzen verbessert.

Was die Zusammensetzung anbelangt, so kann diese bei einer Temperatur von 20 °C eine dynamische Viskosität von 1.000 bis 12.000 mPas, insbesondere 2.000 bis 10.000 mPas, vorzugsweise 4.000 bis 9.000 mPas, bevorzugt 6.000 bis 8.000 mPas, aufweisen. Aufgrund der erfindungsgemäß vorgesehenen Viskosität ist eine besonders einfache und wenig schmerzhafte Instillation der Zusammensetzung in die Harnblase gewährleistet. Weiterhin liegt aufgrund der erfindungsgemäß vorgesehenen Viskosität eine besonders gute Interaktion der Zusammensetzung nach der Erfindung mit dem Urothel vor, was die Wirksamkeit positiv beeinflußt, insbesondere was die Bioverfügbarkeit von Oxybutynin anbelangt.

Gemäß einer erfindungsgemäß besonders bevorzugten Ausführungsform ist die Zusammensetzung der vorliegenden Erfindung, welche vorzugsweise in Form einer wäßrigen Lösung und/oder einer wäßrigen Suspension vorliegt, durch die folgende Rezeptur gekennzeichnet, wobei alle nachstehend genannten Mengenangaben jeweils auf das Volumen der erfindungsgemäßen Zusammensetzung bezogen sind, wobei die Rezeptur in Kombination enthält:
- Oxybutynin und/oder dessen Salze, vorzugsweise Oxybutyninhydrochlorid, insbesondere mit einem Wirkstoffgehalt von 0,01 bis 0,2 mg/ml, insbesondere 0,01 bis 0,15 mg/ml, vorzugsweise 0,02 bis 0,1 mg/ml, bevorzugt 0,03 bis 0,08 mg/ml, besonders bevorzugt 0,04 bis 0,07 mg/ml, berechnet als Oxybutynin,
- Hyaluronsäure und/oder deren Salze, insbesondere nichttierischen Ursprungs, vorzugsweise Natriumhyaluronat, bevorzugt Natriumhyaluronat nichttierischen Ursprungs, insbesondere mit einer mittleren Molmasse (Molekulargewicht) von weniger als 200 kDa, insbesondere mit einer Molmasse im Bereich von 50 bis 195 kDa, insbesondere 100 bis 195 kDa, vorzugsweise 150 bis 190 kDa, bevorzugt 175 bis 190 kDa, und/oder insbesondere mit einem Wirkstoffgehalt von 0,2 bis 2,0 mg/ml, insbesondere 0,3 bis 1,6 mg/ml, vorzugsweise 0,4 bis 1,2 mg/ml, bevorzugt 0,5 bis 1,0 mg/ml, besonders bevorzugt 0,7 bis 0,9 mg/ml, berechnet als Hyaluronsäure und
- gegebenenfalls mindestens einen Elektrolyten, vorzugsweise Natriumchlorid, insbesondere mit einem Gehalt von 0,5 bis 20 mg/ml, insbesondere 1 bis 10 mg/ml, vorzugsweise 4 bis 8 mg/ml,
insbesondere wobei das Mengenverhältnis (Gewichtsverhältnis) von Oxybutynin und/oder dessen Salzen, vorzugsweise Oxybutyninhydrochlorid, einerseits zu Hyaluronsäure und/oder deren Salzen, vorzugsweise Natriumhyaluronat, andererseits in der Zusammensetzung im Bereich von 1 : 1 bis 1 : 200; insbesondere 1 : 2 bis 1 : 160, vorzugsweise 1 : 4 bis 1 : 60, bevorzugt 1 : 6 bis 1 : 35, besonders bevorzugt 1 : 10 bis 1 : 20, ganz besonders bevorzugt bei etwa 1 : 14 liegt, berechnet als Oxybutynin zu Hyaluronsäure.

Gemäß einer bevorzugten Ausführungsform der vorliegenden Erfindung enthalten 50 ml der erfindungsgemäßen Zusammensetzung:

| | |
|---|---|
| Natriumhyaluronat | 40 mg |
| Lösung von Oxybutyninchlorid mit 0,025 Gew.-% Oxybutyninhydrochlorid | 12,5 mg |
| Natriumchlorid | 345 mg |

in vorzugsweise wäßriger Lösung.

Wie zuvor erörtert, eignet sich die erfindungsgemäße Zusammensetzung zur prophylaktischen oder therapeutischen Behandlung von in Zusammenhang mit einer Hyperaktivität und/oder Hyperreflexie des Detrusorapparates (Detrusorhyperaktivität und/oder Detrusorhyperreflexie) stehender Harninkontinenz. Die zu behandelnden Harninkontinenz kann dabei insbesondere auf eine idiopathische und/oder neurogene Detrusorhyperaktivität zurückgeführt werden, wobei diesbezüglich insbesondere die Dranginkontinenz, insbesondere die motorische Dranginkontinenz, zu nennen ist. Gleichermaßen eignet sich die erfindungsgemäße Zusammensetzung auch zur prophylaktischen oder therapeutischen Behandlung der Reflexinkontinenz, wobei diesbezüglich insbesondere die spinale Reflexinkontinenz und die supraspinale Reflexinkontinenz zu nennen ist, wobei diesbezüglich insbesondere eine ungehemmte neuropatische Blase, insbesondere mit einer Detrusorhyperaktivität vorliegt.

Im Rahmen der Anwendung der erfindungsgemäßen Zusammensetzung kann die Zusammensetzung nach der Erfindung zur Instillation und/oder zur vorzugsweise topischen Applikation in den Urogenitalbereich, insbesondere in die Harnblase, vorgesehen sein.

Was die Zusammensetzung nach der Erfindung weiterhin anbelangt, so ist diese vorzugsweise dosierfertig in Behältnisse eingebracht, wobei diesbezüglich insbesondere Volumengrößen von 3 bis 70 ml, insbesondere 40 bis 60 ml, bevorzugt etwa 50 ml, im Behältnis bzw. Applikationseinheit bzw. Dosiereinheit vorgesehen sein können.

Die vorliegende Erfindung betrifft - gemäß einem **weiteren** Aspekt der vorliegenden Erfindung - gleichermaßen ein Behältnis, welches die erfindungsgemäße Zusammensetzung enthält. Das erfindungsgemäße Behältnis, welches die Zusammensetzung nach der Erfindung enthält, kann insbesondere in Form einer Durchstechflasche, vorzugsweise mit sterilem Verschluß, vorliegen.

Gemäß einem wiederum **weiteren** Aspekt betrifft die vorliegende Erfindung gleichermaßen eine Applikationsvorrichtung zur Instillation bzw. zur topischen Applikation der erfindungsgemäßen Zusammensetzung in den Urogenitalbereich, insbesondere in die Harnblase. Die erfindungsgemäße Applikationsvorrichtung enthält die erfindungsgemäße Zusammensetzung. Bei der erfindungsgemäßen Applikationsvorrichtung kann es sich insbesondere um eine Applikationsvorrichtung zur einmaligen Anwendung handeln, welche in nichtbeschränkender Weise ein Aufnahme- bzw. Vorratsbehältnis zur Aufnahme der erfindungsgemäßen Zusammensetzung sowie eine Applikationseinrichtung, beispielsweise einen Instillationsschlauch bzw. einen Katheter oder dergleichen, enthält. Bei der Applikationsvorrichtung kann es sich beispielsweise um eine vorzugsweise sterile Spritze handeln, welche die erfindungsgemäße Zusammensetzung enthält. Die erfindungsgemäße Spritze kann mit einer Applikationseinrichtung, beispielsweise mit einem Instillationsschlauch bzw. einem Katheter oder dergleichen, ausgestattet sein bzw. mit diesem verbunden sein, insbesondere um eine Instillation bzw. Applikation der Zusammensetzung nach der Erfindung in die Harnblase zu ermöglichen. Die erfindungsgemäße Applikationsvorrichtung kann vorzugsweise nach Art einer Einmaldosiervorrichtung bzw. Einmalapplikationsvorrichtung konzipiert sein.

Weiterer Gegenstand der vorliegenden Erfindung - gemäß einem noch **weiteren** Aspekt der vorliegenden Erfindung - ist die Verwendung der Zusammensetzung nach der vorliegenden Erfindung zur prophylaktischen oder therapeutischen Behandlung von Erkrankungen des Urogenitaltraktes, insbesondere von Harninkontinenz, vorzugsweise von in Zusammenhang mit einer Hyperaktivität und/oder Hyperreflexie des Detrusorapparates (Detrusorhyperaktivität und/oder Detrusorhyperreflexie) stehender Harninkontinenz.

Wie zuvor ausgeführt, kann es sich bei der in Rede stehenden Harninkontinenz um eine durch idiopathische und/oder neurogene Detrusorhyperaktivität und/oder Detrusorhyperreflexie hervorgerufene Harninkontinenz handeln. Dies kann beispielsweise eine motorische Dranginkontinenz oder eine insbesondere spinale oder supraspinale Reflexinkontinenz sein. Mit anderen Worten zielt die vorliegende Erfindung insbesondere auf die prophylaktische oder therapeutische Behandlung von Harninkontinenzerkrankungen ab, die in Zusammenhang mit einer übermäßigen Aktivität des Detrusormuskels stehen.

Dabei führt die Verwendung der erfindungsgemäßen Zusammensetzung zu einer deutlichen Linderung bzw. Ausheilung der Erkrankung. Dabei ist erfindungsgemäß aufgrund der Verwendung zweier Wirksubstanzen gewissermaßen eine Doppelstrategie hinsichtlich der zu behandelnden Inkontinenzerkrankungen realisiert worden: Denn der Einsatz von Oxybutynin führt zu einer Relaxation bzw. Entspannung der glatten Blasenmuskulatur und somit insbesondere des Detrusors, da es auf postganglionäre Muskarinrezeptoren im Sinne eines kompetetiven Antagonisten von Acetylcholin wirkt. Hierdurch wird die Detrusorkontraktion vermindert und das Harndranggefühl setzt später ein, d. h. bei einem höheren Füllungsvolumen der Harnblase. Die Verwendung von Hyaluronsäure in der erfindungsgemäßen Zusammensetzung führt - neben dem positiven Effekt auf das Urothel - zu einer deutlichen Steigerung der Wirkung des Oxybutynins, insbesondere was dessen Langzeitwirkung anbelangt, denn das Oxybutynin wird - ohne sich hierauf festlegen zu wollen - gewissermaßen in eine Hyaluronsäurematrix eingelagert. Dies führt zu einem Controlled-Release-Effekt, wodurch die Bioverfügbarkeit der Wirksubstanzen insgesamt signifikant gesteigert ist. Zudem führt die Hyaluronsäure zu einer Regeneration des Urothels, so daß auch hierdurch die Schmerzsymptomatik verbessert wird.

Darüber hinaus kann die erfindungsgemäße Zusammensetzung auch zur prophylaktischen und/oder therapeutischen Behandlung von Erkrankungen des Urogenitaltraktes, insbesondere von entzündlichen Erkrankungen der Harnblase, vorzugsweise von Cystitis, bevorzugt von interstitieller Cystitis, radiogener Cystitis und/oder chronifizierter bakterieller Cystitis, besonders bevorzugt von interstitieller Cystitis, verwendet werden.

Zu Zwecken der erfindungsgemäßen Verwendung wird die erfindungsgemäße Zusammensetzung - wie zuvor beschrieben - in den Urogenitaltrakt, insbesondere in die Harnblase instilliert bzw. vorzugsweise topisch appliziert. Dabei sollte die Instillation bzw. die vorzugsweise topische Applikation in die vorzugsweise entleerte Harnblase erfolgen. Diesbezüglich sollte die Zusammensetzung nach der Erfindung in der Harnblase für einen Zeitraum von 30 bis 120 Minuten verbleiben, um eine optimale Einwirkung der Wirkstoffe zu ermöglichen. Folglich sollte - genauer gesagt - das gesamte Volumen der in dem erfindungsgemäßen Behältnis bzw. in der erfindungsgemäßen Applikationsvorrichtung vorhandenen Zusammensetzung nach der Erfindung, vorzugsweise 50 ml, nach vollständiger Entleerung in die Harnblase instilliert werden. Um optimale Ergebnisse zu erreichen, sollte die Zusammensetzung nach der Erfindung möglichst lange in der Harnblase verbleiben, und zwar - wie zuvor beschrieben - für einen Zeitraum von 30 Minuten bis maximal etwa 2 Stunden. Im Rahmen der Behandlung der Harninkontinenz ist es empfehlenswert, die Instillation der erfindungsgemäßen Zusammensetzung für einen bestimmten Zeitraum, beispielsweise für einen Zeitraum von vier Wochen, jeweils ein- bis siebenmal, insbesondere ein- bis dreimal pro Woche durchzuführen, wobei die diesbezügliche Dosis 50 ml pro Anwendung betragen sollte. Im Rahmen einer Erhaltungstherapie kann die erfindungsgemäße Zusammensetzung anschließend beispielsweise einmal pro Monat angewendet werden.

Die vorliegende Erfindung weist gegenüber dem Stand der Technik zahlreiche Vorteile auf, welche nachfolgend beispielhaft aufgeführt sind:
- Durch die gleichzeitige Verwendung von Oxybutynin und Hyaluronsäure in der erfindungsgemäßen Zusammensetzung wird gewissermaßen eine Doppelstrategie realisiert:
   - Durch die Verwendung von Oxybutynin erfolgt eine effektive Relaxation bzw. Erschlaffung insbesondere des Detrusors, so daß das Harndranggefühl bzw. die einhergehende Harninkontinenz signifikant verringert bzw. unterbunden wird. Zudem wird das Füllvolumen der Harnblase erhöht.
   - Die Verwendung einer Hyaluronsäure führt auch zu einer besseren Bioverfügbarkeit in bezug auf die Wirksubstanz Oxybutynin, da - ohne sich auf diese Theorie festlegen zu wollen - das Oxybutynin gewissermaßen in die Matrix aus Hyaluronsäure, auch in bezug auf das regenerierte Urothel, eingelagert wird und somit gewissermaßen eine bessere Langzeitverfügbarkeit im Sinne eines Controlled-Release-Effektes vorliegt. Hierdurch kann die Konzentration bzw. Dosis in bezug auf Oxybutynin weiter verringert werden. Der Einsatz von Hyaluronsäure führt zu einer effektiven Regeneration des bei Harninkontinenz oftmals geschädigten Urothels, was zu einer deutlichen Verminderung von Schmerzen führt.
- Aufgrund der intravesikalen Instillation der erfindungsgemäßen Zusammensetzung kann eine systemische Verabreichung vermieden werden. Die Wirksubstanzen liegen direkt am Wirkort vor, wobei - ohne sich auf diese Theorie beschränken zu wollen - das Oxybutynin von dem Blasengewebe resorbiert wird und somit nahezu direkt vom Applikationsort auf die Detrusormuskulatur einwirken kann.
- Durch die intravesikale Applikation kann der Wirkstoffgehalt bzw. die Dosis ein Oxybutynin deutlich verringert werden. Zudem wird eine systemische Verabreichung über den Gastrointestinaltrakt einhergehend mit zusätzlichen Resorptions- und Stoffwechselverlusten vermieden.
- Durch die intravesikale Applikation einerseits und die gezielte Kombination von Oxybutynin und Hyaluronsäure andererseits treten die für die kompetitive Acetylcholinantagonisten typischen Nebenwirkungen, insbesondere wie zuvor erwähnt, nicht bzw. nur stark abgeschwächt auf. Die erfindungsgemäße Zusammensetzung ist daher außerordentlich gut verträglich, da Nebenwirkungen bei gleichzeitig hoher Wirksamkeit praktisch nicht auftreten.
- Durch die Regeneration des Urothels werden die Schmerzsymptome signifikant verringert. Gleichzeitig wird durch das Oxybutynin durch die Einwirkung auf den Detrusor die Harninkontinenz wirksam verringert bzw. unterbunden, wobei sowohl die Frequenz des Harnlassens als auch das maximale Fassungsvermögen der Harnblase bis zur ersten Detrusorkontraktion signifikant erhöht ist. Somit wird der Harndrang und die Frequenz von Inkontinenzepisoden bzw. von willkürlicher Blasenentleerung deutlich verlängert.
- Im Rahmen der erfindungsgemäßen Zusammensetzung liegt ein synergistischer Effekt vor, da die Wirkung der Zusammensetzung über die Wirkung der Einzelkomponenten hinausgeht, was insbesondere durch die bessere Bioverfügbarkeit bei verringerter Konzentration an Oxybutynin, bedingt durch die Verwendung von Hyaluronsäure, hervorgerufen wird.
- Mit der erfindungsgemäßen Zusammensetzung behandelte Patienten wiesen bereits nach wenigen Behandlungen einen signifikant verbesserten Gesundheitsstatus auf, einhergehend mit einer deutlichen Verringerung der Schmerzsymptomatik und der Harninkontinenz.
- Darüber hinaus ist durch die Verwendung von Hyaluronsäure bzw. deren Salze mit einem Molekulargewicht von weniger als 200 kDa die Herstellung der erfindungsgemäßen Zusammensetzung verbessert, da eine stabilere Lösung bzw. Suspension realisiert werden kann, so daß auch deren Lagerfähigkeit verbessert ist. Zudem kann die erfindungsgemäße Zusammensetzung beispielsweise aufgrund der verbesserten Viskosität einfach instilliert bzw. topisch appliziert werden.

### Ausführungsbeispiele:

Im Rahmen von seitens der Anmelderin durchgeführten Ausführungsbeispielen bzw. Wirksamkeitsstudien wurde die herausragende Wirkung einer Zusammensetzung nach der vorliegenden Erfindung auf Basis der erfindungsgemäßen Wirkstoffkombination (vgl. nachfolgende "Zusammensetzung C") gegenüber Vergleichzusammensetzung (vgl. nachfolgende "Zusammensetzung B") und einer Blindprobe ("Placebo, Zusammensetzung A") belegt. In diesem Zusammenhang wurde die verbesserte Wirksamkeit der erfindungsgemäßen binären Kombination auf Basis von Oxybutynin einerseits und Hyaluronsäure andererseits gegenüber der Einzelsubstanz Oxybutynin belegt.

Die hervorragende Wirksamkeit der erfindungsgemäßen Kombination wurde anhand von verschiedenen harnblasenspezifischen Parametern - wie in der nachfolgenden Tabelle angeführt - ermittelt. Alle Zusammensetzungen sind auf Basis einer Natriumchloridlösung formuliert und enthalten die folgenden Wirkstoffe:

### Zusammensetzung A (Placebo):

### Reine Natriumchloridlösung (Osmolarität entsprechend der von Urin)

| Zusammensetzung B (Vergleich): | |
|---|---|
| Oxybutyninhydrochlorid | 3,125 mg pro 50 ml |

| Zusammensetzung C (Erfindung): | |
|---|---|
| Oxybutyninhydrochlorid | 3,125 mg pro 50 ml |
| Natriumhyaluronat nichttierischen Ursprungs | |
| (M = 180 kDa) | 40 mg pro 50 ml |

Im Rahmen der Untersuchungen wurden insgesamt 55 Frauen mit diagnostizierter idiopathischer Dranginkontinenz einer Instillationsbehandlung mit den zuvor genannten Zusammensetzungen unterzogen. Die jeweilige Größe der Untersuchungsgruppen betrug n = 15 für die Zusammensetzung A und jeweils n = 20 für die Zusammensetzungen B und C. Die Zusammensetzungen wurden über einen Zeitraum von 15 Tagen mit einem Abstand von zwei Tagen in die Harnblase instilliert. Das Volumen der instillierten Lösung betrug in allen Fällen jeweils 50 ml. Vor der ersten Instillation (Zeitpunkt t = 0) und nach Beendigung des Versuches (Zeitpunkt t = 15 Tage) wurden Miktionsprotokolle und urodynamische Untersuchungen durchgeführt. Die Ergebnisse sind in der nachfolgenden Tabelle angeführt:

**Tabelle:**

| | Zusammensetzung | | Zusammensetzung | | Zusammensetzung | |
|---|---|---|---|---|---|---|
| | A | | B | | C | |
| | t = 0 | t = 15 d | t = 0 | t = 15 d | t = 0 | t = 15 d |
| Miktion pro Tag | 9,2 ± 1,7 | 8,6 ± 1,1 | 8,2 ± 1,6 | 6,4 ± 1,0 | 10,0 ± 2,3 | 3,8 ± 1,3 |
| Harndrang bei Harnvolumen (ml) | 85,6 ± 17,8 | 90,1 ± 22,8 | 90,7 ± 15,4 | 120,3 ± 26,8 | 92,5 ± 25,4 | 148,3 ± 19,7 |
| Blasenkapazität (ml) | 170,5 ± 40,8 | 185,1 ± 45,7 | 182,4 ± 52,1 | 254,1 ± 41,1 | 165,5 ± 59,8 | 295,4 ± 55,0 |
| Motorische Detrusoraktivität | 13 | 11 | 14 | 8 | 12 | 4 |
| in der Blase verbleibender Restharn (ml) | 25,4 ± 5,6 | 30,5 ± 7,1 | 30,1 ± 10,3 | 70,5 ± 14,4 | 27,8 ± 6,2 | 76,7 ± 12,9 |

Die Tabelle veranschaulicht die hervorragende Wirkung der erfindungsgemäßen Zusammensetzung auf Basis der binären Kombination von Oxybutyninhydrochlorid einerseits und Natriumhyaluronat andererseits gemäß Zusammensetzung C gegenüber Placebo (Zusammensetzung A) und der Vergleichzusammensetzung B, welche nur Oxybutyninhydrochlorid enthält. Die Anzahl der Miktionen pro Tag nahm in bezug auf die erfindungsgemäße Kombination C gegenüber der Vergleichzusammensetzung B nochmals deutlich ab, und der Harndrang setzte bei den Probanden der Untersuchungsgruppe, die mit der erfindungsgemäßen Zusammensetzung C behandelt wurden, nochmals deutlich später ein, d. h. bei einem größeren Harnvolumen in der Blase, als bei Placebo und der Untersuchungsgruppe, die das Monopräparat gemäß Zusammensetzung B erhielten. Damit einher ging eine signifikante Erhöhung der Blasenkapazität und eine Abnahme der motorischen Detrusoraktivität bei den Probanden, welche die erfindungsgemäße Zusammensetzung C erhielten, im Vergleich zu der Untersuchungsgruppe mit der Vergleichszusammensetzung B. Schließlich konnte in bezug auf die erfindungsgemäße Zusammensetzung C eine im Vergleich zu der Zusammensetzung A und B nochmals gesteigerte Restharnmenge ermittelt werden.

Dieser Test belegt somit die besonders gute Wirkung der erfindungsgemäßen Kombination gemäß Zusammensetzung C im Vergleich zur Kontrolluntersuchung (Placebo) gemäß Versuchsgruppe A sowie gegenüber der anderen Vergleichszusammensetzung B.

Die vorstehende Versuchsreihe zeigt insgesamt die hervorragende, synergistische Wirkung der erfindungsgemäßen binären Kombination gegenüber den jeweiligen Kontrollexperimenten.

## Patentansprüche

1. Zusammensetzung, insbesondere pharmazeutische Zusammensetzung, vorzugsweise zur Behandlung von Erkrankungen des Urogenitaltraktes, bevorzugt von Harninkontinenz, enthaltend in Kombination und jeweils in pharmazeutisch wirksamen Mengen
(a) Oxybutynin und/oder dessen Salze sowie
(b) Hyaluronsäure und/oder deren Salze, wobei die Hyaluronsäure und/oder deren Salze eine mittlere Molmasse (Molekulargewicht) von weniger als 200 kDa aufweist.

2. Zusammensetzung nach Anspruch 1, enthaltend (a) Oxybutynin und/oder dessen Salze in Mengen von 0,001 bis 0,02 Gew.-%, insbesondere 0,001 bis 0,015 Gew.-%, vorzugsweise 0,002 bis 0,01 Gew.-%, bevorzugt 0,003 bis 0,008 Gew.-%, besonders bevorzugt 0,004 bis 0,007 Gew.-%, bezogen auf das Gesamtgewicht der Zusammensetzung und berechnet als Oxybutynin, und/oder enthaltend (a) Oxybutynin in Form eines insbesondere sauren Salzes, vorzugsweise in Form eines sauren Additionssalzes, bevorzugt in Form eines Hydrochlorids.

3. Zusammensetzung nach Anspruch 1 oder 2, wobei (b) die Hyaluronsäure und/oder deren Salze eine mittlere Molmasse (Molekulargewicht) im Bereich von 50 bis 195 kDa, vorzugsweise 100 bis 195 kDa, bevorzugt 150 bis 190 kDa, besonders bevorzugt 175 bis 190 kDa, aufweist und/oder wobei die Zusammensetzung (b) die Hyaluronsäure und/oder deren Salze in Mengen von 0,02 bis 0,2 Gew.-%, insbesondere 0,03 bis 0,16 Gew.-%, vorzugsweise 0,04 bis 0,12 Gew.-%, bevorzugt 0,05 bis 0,10 Gew.-%, bezogen auf das Gesamtgewicht der Zusammensetzung und berechnet als Hyaluronsäure, enthält.

4. Zusammensetzung nach einem oder mehreren der vorangehenden Ansprüche, wobei (b) die Hyaluronsäure und/oder deren Salze nichttierischen Ursprungs ist und/oder wobei die Hyaluronsäure und/oder deren Salze bakteriellen Ursprungs ist, insbesondere wobei die Hyaluronsäure und/oder deren Salze vorzugsweise fermentativ von Bakterien der Gattung *Streptococcus*, insbesondere *Streptococcus lancefields*, bevorzugt *Streptococcus lancefields* Stamm A, gewonnen ist, und/oder wobei die Zusammensetzung als Komponente (b) mindestens ein Alkalihyaluronat, vorzugsweise Natriumhyaluronat, enthält.

5. Zusammensetzung nach einem oder mehreren der vorangehenden Ansprüche, enthaltend außerdem (c) mindestens einen Elektrolyten, insbesondere in Mengen von 0,005 Gew.-% bis 1,5 Gew.-%, insbesondere 0,05 bis 1,25 Gew.-%, vorzugsweise 0,25 bis 1 Gew.-%, bevorzugt 0,5 bis 0,75 Gew.-%, bezogen auf das Gesamtgewicht der Zusammensetzung, insbesondere wobei der mindestens eine Elektrolyt ausgewählt ist aus organischen und/oder anorganischen Alkali- oder Erdalkalisalzen sowie deren Mischungen, vorzugsweise aus anorganischen Alkali- oder Erdalkalisalzen, insbesondere wobei das anorganische Alkali- oder Erdalkalisalz ein Chlorid, vorzugsweise Natriumchlorid, ist.

6. Zusammensetzung nach einem oder mehreren der vorangehenden Ansprüche, enthaltend außerdem (d) Chondroitinsulfat, insbesondere in Mengen von 0,02 bis 0,2 Gew.%, vorzugsweise 0,03 bis 0,16 Gew.%, bevorzugt 0,04 bis 0,12 Gew.-%, besonders bevorzugt 0,05 bis 0,10 Gew.-%, bezogen auf das Gesamtgewicht der Zusammensetzung, und/oder enthaltend außerdem (e) Dexpanthenol und/oder dessen Derivate, insbesondere in Mengen von 0,0001 bis 5 Gew.-%, vorzugsweise 0,001 bis 4 Gew.-%, bevorzugt 0,01 bis 3 Gew.%, besonders bevorzugt 0,1 bis 2 Gew.-%, bezogen auf das Gesamtgewicht der Zusammensetzung, und/oder enthaltend außerdem (f) Ectoin und/oder dessen Derivate, insbesondere in Mengen von 0,0001 bis 5 Gew.%, vorzugsweise 0,001 bis 4 Gew.-%, bevorzugt 0,01 bis 3 Gew.-%, besonders bevorzugt 0,1 bis 2 Gew.%, bezogen auf das Gesamtgewicht der Zusammensetzung.

7. Zusammensetzung nach einem oder mehreren der vorangehenden Ansprüche, enthaltend außerdem (g) mindestens ein Lokalanästhetikum, insbesondere in Mengen von 0,0001 bis 5 Gew.-%, vorzugsweise 0,001 bis 4 Gew.-%, bevorzugt 0,01 bis 3 Gew.-%, besonders bevorzugt 0,1 bis 2 Gew.-%, bezogen auf das Gesamtgewicht der Zusammensetzung, und/oder enthaltend außerdem (h) Mineralstoffe und/oder Vitamine, insbesondere Zinkverbindungen, vorzugsweise organische Zinkverbindungen, bevorzugt Zinkgluconat, insbesondere in Mengen von 0,0001 bis 5 Gew.-%, vorzugsweise 0,001 bis 4 Gew.-%, bevorzugt 0,01 bis 3 Gew.-%, besonders bevorzugt 0,1 bis 2 Gew.-%, bezogen auf das Gesamtgewicht der Zusammensetzung.

8. Zusammensetzung nach einem oder mehreren der vorangehenden Ansprüche, wobei die Zusammensetzung in flüssiger Form, insbesondere in Form einer wäßrigen Lösung oder einer wäßrigen Suspension, vorliegt und/oder wobei die Zusammensetzung einen pH-Wert von 5,5 bis 7,5, insbesondere 6,0 bis 7,0, vorzugsweise 6,2 bis 6,8, aufweist und/oder wobei die Zusammensetzung bei einer Temperatur von 20 °C eine dynamische Viskosität von 1.000 bis 12.000 mPas, insbesondere 2.000 bis 10.000 mPas, vorzugsweise 4.000 bis 9.000 mPas, bevorzugt 6.000 bis 8.000 mPas, aufweist.

9. Zusammensetzung nach einem oder mehreren der vorangehenden Ansprüche, insbesondere in Form einer wäßrigen Lösung oder einer wäßrigen Suspension, enthaltend in Kombination
(a) Oxybutynin und/oder dessen Salze, vorzugsweise Oxybutyninhydrochlorid, insbesondere mit einem Wirkstoffgehalt von 0,01 bis 0,2 mg/ml, insbesondere 0,01 bis 0,15 mg/ml, vorzugsweise 0,02 bis 0,1 mg/ml, bevorzugt 0,03 bis 0,08 mg/ml, besonders bevorzugt 0,04 bis 0,07 mg/ml, bezogen auf das Volumen der Zusammensetzung und berechnet als Oxybutynin,
(b) Hyaluronsäure und/oder deren Salze, insbesondere nichttierischen Ursprungs, vorzugsweise Natriumhyaluronat, bevorzugt Natriumhyaluronat nichttierischen Ursprungs, mit einer mittleren Molmasse (Molekulargewicht) von weniger als 200 kDa, insbesondere mit einer Molmasse im Bereich von 50 bis 195 kDa, insbesondere 100 bis 195 kDa, vorzugsweise 150 bis 190 kDa, bevorzugt 175 bis 190 kDa, und/oder insbesondere mit einem Wirkstoffgehalt von 0,2 bis 2,0 mg/ml, insbesondere 0,3 bis 1,6 mg/ml, vorzugsweise 0,4 bis 1,2 mg/ml, bevorzugt 0,5 bis 1,0 mg/ml, besonders bevorzugt 0,7 bis 0,9 mg/ml, bezogen auf das Volumen der Zusammensetzung und berechnet als Hyaluronsäure, und
(c) gegebenenfalls mindestens einen Elektrolyten, vorzugsweise Natriumchlorid, insbesondere mit einem Gehalt von 0,5 bis 20 mg/ml, insbesondere 1 bis 10 mg/ml, vorzugsweise 4 bis 8 mg/ml,
insbesondere wobei das Mengenverhältnis (Gewichtsverhältnis) von (a) Oxybutynin und/oder dessen Salzen, vorzugsweise Oxybutyninhydrochlorid, einerseits zu (b) Hyaluronsäure und/oder deren Salzen, vorzugsweise Natriumhyaluronat, andererseits in der Zusammensetzung im Bereich von 1 : 1 bis 1 : 200, insbesondere 1 : 2 bis 1 : 160, vorzugsweise 1 : 4 bis 1: 60, bevorzugt 1 : 6 bis 1 : 35, besonders bevorzugt 1 : 10 bis 1 : 20, ganz besonders bevorzugt bei etwa 1 : 14, liegt, berechnet als Oxybutynin zu Hyaluronsäure.

10. Zusammensetzung nach einem oder mehreren der vorangehenden Ansprüche zur Instillation und/oder zur vorzugsweise topischen Applikation in den Urogenitalbereich, insbesondere in die Harnblase.

11. Behältnis, enthaltend eine Zusammensetzung nach einem oder mehreren der vorangehenden Ansprüche, insbesondere in Form einer Durchstichflasche mit vorzugsweise sterilem Verschluß.

12. Applikationsvorrichtung zur Instillation und/oder zur vorzugsweise topischen Applikation in den Urogenitalbereich, insbesondere in die Harnblase, enthaltend eine Zusammensetzung nach einem der Ansprüche 1 bis 10.

13. Verwendung einer Zusammensetzung nach einem der Ansprüche 1 bis 10 zur Herstellung eines Arzneimittels zur prophylaktischen oder therapeutischen Behandlung von Erkrankungen des Urogenitaltraktes, insbesondere von Harninkontinenz, vorzugsweise von in Zusammenhang mit einer Hyperaktivität und/oder Hyperreflexie des Detrusorapparates (Detrusorhyperaktivität und/oder Detrusorhyperreflexie) stehender Harninkontinenz, insbesondere wobei sie Zusammensetzung zur Herstellung eines Arzneimittels zur prophylaktischen oder therapeutischen Behandlung von durch idiopathischer und/oder neurogener Detrusorhyperaktivität und/oder Detrusorhyperreflexie hervorgerufener Harninkontinenz verwendet wird.

14. Verwendung nach Anspruch 13 zur Herstellung eines Arzneimittels zur prophylaktischen oder therapeutischen Behandlung von motorischer Dranginkontinenz und/oder insbesondere spinaler und/oder insbesondere supraspinaler Reflexinkontinenz.

15. Verwendung einer Zusammensetzung nach einem der Ansprüche 1 bis 10 zur Herstellung eines Arzneimittels zur Behandlung von Erkrankungen des Urogenitaltraktes, insbesondere von entzündlichen Erkrankungen der Harnblase, vorzugsweise von Cystitis, bevorzugt von interstitieller Cystitis, radiogener Cystitis und/oder chronifizierter bakterieller Cystitis, besonders bevorzugt von interstitieller Cystitis.

## Claims

1. Composition, particularly a pharmaceutical composition, preferably for treating diseases of the urogenital tract, more preferably for treating urinary incontinence, containing a combination of the following, each in pharmaceutically active amounts:
(a) oxybutynin and/or salts thereof, and
(b) hyaluronic acid and/or salts thereof, wherein the hyaluronic acid and/or salts thereof has/have a mean molar mass (molecular weight) of less than 200 kDa.

2. Composition according to Claim 1, containing (a) oxybutynin and/or salts thereof in amounts of 0.001 to 0.02 % by weight, particularly 0.001 to 0.015 % by weight, preferably 0.002 to 0.01 % by weight, more preferably 0.003 to 0.008 % by weight, particularly preferably 0.004 to 0.007 % by weight, relative to the total weight of the composition and calculated as oxybutynin, and/or containing (a) oxybutynin particularly in the form of an acid salt, preferably in the form of an acid addition salt, more preferably in the form of a hydrochloride.

3. Composition according to Claim 1 or 2, wherein (b) the hyaluronic acid and/or salts thereof has/have a mean molar mass (molecular weight) in the range of 50 to 195 kDa, preferably 100 to 195 kDa, more preferably 150 to 190 kDa, particularly preferably 175 to 190 kDa, and/or wherein the composition contains (b) the hyaluronic acid and/or salts thereof in amounts of 0.02 to 0.2 % by weight, particularly 0.03 to 0.16 % by weight, preferably 0.04 to 0.12 % by weight, more preferably 0.05 to 0.10 % by weight, relative to the total weight of the composition and calculated as hyaluronic acid.

4. Composition according to one or more of the preceding claims, wherein (b) the hyaluronic acid and/or salts thereof is/are of non-animal origin, and/or wherein the hyaluronic acid and/or salts thereof is/are of bacterial origin, particularly wherein the hyaluronic acid and/or salts thereof is/are recovered preferably by fermentation from bacteria of the genus *Streptococcus*, particularly *Streptococcus lancefields,* preferably *Streptococcus lancefields* strain A, and/or wherein the composition contains as component (b) at least one alkali hyaluronate, preferably sodium hyaluronate.

5. Composition according to one or more of the preceding claims, additionally containing (c) at least one electrolyte, particularly in amounts of 0.005 % by weight to 1.5 % by weight, particularly 0.05 to 1.25 % by weight, preferably 0.25 to 1 % by weight, more preferably 0.5 to 0.75 % by weight, relative to the total weight of the composition, particularly wherein the at least one electrolyte is chosen from organic and/or inorganic alkaline salts or alkaline earth salts and mixtures thereof, preferably from inorganic alkaline salts or alkaline earth salts, particularly wherein the inorganic alkaline salt or alkaline earth salt is a chloride, preferably sodium chloride.

6. Composition according to one or more of the preceding claims, additionally containing (d) chondroitin sulphate, particularly in amounts of 0.02 to 0.2 % by weight, preferably 0.03 to 0.16 % by weight, more preferably 0.04 to 0.12 % by weight, particularly preferably 0.05 to 0.10 % by weight, relative to the total weight of the composition, and/or additionally containing (e) dexpanthenol and/or derivatives thereof, particularly in amounts of 0.0001 to 5 % by weight, preferably 0.001 to 4 % by weight, more preferably 0.01 to 3 % by weight, particularly preferably 0.1 to 2 % by weight, relative to the total weight of the composition, and/or additionally containing (f) ectoin and/or derivatives thereof, particularly in amounts of 0.0001 to 5 % by weight, preferably 0.001 to 4 % by weight, more preferably 0.01 to 3 % by weight, particularly preferably 0.1 to 2 % by weight, relative to the total weight of the composition.

7. Composition according to one or more of the preceding claims, additionally containing (g) at least one local anaesthetic, particularly in amounts of 0.0001 to 5 % by weight, preferably 0.001 to 4 % by weight, more preferably 0.01 to 3 % by weight, particularly preferably 0.1 to 2 % by weight, relative to the total weight of the composition, and/or additionally containing (h) minerals and/or vitamins, particularly zinc compounds, preferably organic zinc compounds, more preferably zinc gluconate, particularly in amounts of 0.0001 to 5 % by weight, preferably 0.001 to 4 % by weight, more preferably 0.01 to 3 % by weight, particularly preferably 0.1 to 2 % by weight relative to the total weight of the composition.

8. Composition according to one or more of the preceding claims, wherein the composition is present in liquid form, particularly in the form of an aqueous solution or an aqueous suspension, and/or wherein the composition has a pH value of 5.5 to 7.5, particularly 6.0 to 7.0, preferably 6.2 to 6.8, and/or wherein the composition at a temperature of 20°C has a dynamic viscosity of 1000 to 12000 mPas, particularly 2000 to 10000 mPas, preferably 4000 to 9000 mPas, more preferably 6000 to 8000 mPas.

9. Composition according to one or more of the preceding claims, particularly in the form of an aqueous solution or an aqueous suspension, containing in combination:
(a) oxybutynin and/or salts thereof, preferably oxybutynin hydrochloride, particularly with an active substance content of 0.01 to 0.2 mg/ml, particularly 0.01 to 0.15 mg/ml, preferably 0.02 to 0.1 mg/ml, more preferably 0.03 to 0.08 mg/ml, particularly preferably 0.04 to 0.07 mg/ml, relative to the volume of the composition and calculated as oxybutynin,
(b) hyaluronic acid and/or salts thereof, particularly of non-animal origin, preferably sodium hyaluronate, more preferably sodium hyaluronate of non-animal origin, with a mean molar mass (molecular weight) of less than 200 kDa, particularly with a molar mass in the range of 50 to 195 kDa, particularly 100 to 195 kDa, preferably 150 to 190 kDa, more preferably 175 to 190 kDa, and/or particularly with an active substance content of 0.2 to 2.0 mg/ml, particularly 0.3 to 1.6 mg/ml, preferably 0.4 to 1.2 mg/ml, more preferably 0.5 to 1.0 mg/ml, particularly preferably 0.7 to 0.9 mg/ml, relative to the volume of the composition and calculated as hyaluronic acid, and
(c) optionally at least one electrolyte, preferably sodium chloride, particularly with a content of 0.5 to 20 mg/ml, particularly 1 to 10 mg/ml, preferably 4 to 8 mg/ml,
particularly wherein the mass ratio (weight ratio) of (a) oxybutynin and/or salts thereof, preferably oxybutynin hydrochloride, to (b) hyaluronic acid and/or salts thereof, preferably sodium hyaluronate, in the composition, is in the range of 1:1 to 1:200, particularly 1:2 to 1:160, preferably 1:4 to 1:60, more preferably 1:6 to 1:35, particularly preferably 1:10 to 1:20, very particularly preferably about 1:14, calculated as oxybutynin to hyaluronic acid.

10. Composition according to one or more of the preceding claims, for instillation and/or for preferably topical application into the urogenital area, particularly into the bladder.

11. Container containing a composition according to one or more of the preceding claims, particularly in the form of a pierceable vial with a preferably sterile closure.

12. Applicator device for instillation and/or for preferably topical application into the urogenital area, particularly into the bladder, containing a composition according to one of Claims 1 to 10.

13. Use of a composition according to one of Claims 1 to 10 for producing a medicament for prophylactic or therapeutic treatment of diseases of the urogenital tract, particularly urinary incontinence, preferably urinary incontinence associated with hyperactivity and/or hyperreflexia of the detrusor apparatus (detrusor hyperactivity and/or detrusor hyperreflexia), particularly wherein the composition is used to produce a medicament for prophylactic or therapeutic treatment of urinary incontinence caused by idiopathic and/or neurogenic detrusor hyperactivity and/or detrusor hyperreflexia.

14. Use according to Claim 13 for producing a medicament for prophylactic or therapeutic treatment of motor urge incontinence and/or particularly spinal and/or particularly supraspinal reflex incontinence.

15. Use of a composition according to one of Claims 1 to 10 for producing a medicament for treating diseases of the urogenital tract, particularly inflammatory diseases of the bladder, preferably cystitis, more preferably interstitial cystitis, radiogenic cystitis and/or chronic bacterial cystitis, particularly preferably interstitial cystitis.

## Revendications

1. Composition, en particulier composition pharmaceutique, de préférence pour le traitement de maladies du tractus urogénital, de préférence de l'incontinence urinaire, contenant en combinaison et à chaque fois en des quantités pharmaceutiquement actives
(a) de l'oxybutynine et/ou ses sels ainsi que
(b) de l'acide hyaluronique et/ou ses sels, où l'acide hyaluronique et/ou ses sels présente(nt) une masse molaire moyenne (poids moléculaire) inférieure à 200 kDa.

2. Composition selon la revendication 1, contenant (a) de l'oxybutynine et/ou ses sels en des quantités de 0,001 à 0,02 % en poids, en particulier de 0,001 à 0,015 % en poids, de préférence de 0,002 à 0,01 % en poids, de préférence de 0,003 à 0,008 % en poids, de manière particulièrement préférée de 0,004 à 0,007 % en poids, par rapport au poids total de la composition et calculé sous forme d'oxybutynine, et/ou contenant (a) de l'oxybutynine sous forme d'un sel en particulier acide, en particulier sous forme d'un sel d'addition d'acide, de préférence sous forme d'un chlorhydrate.

3. Composition selon la revendication 1 ou 2, où (b) l'acide hyaluronique et/ou ses sels présente(nt) une masse molaire moyenne (poids moléculaire) dans la plage de 50 à 195 kDa, de préférence de 100 à 195 kDa, de préférence de 150 à 190 kDa, de manière particulièrement préférée de 175 à 190 kDa et/ou où la composition contient (b) de l'acide hyaluronique et/ou ses sels en des quantités de 0,02 à 0,2 % en poids, en particulier de 0,03 à 0,16 % en poids, de préférence de 0,04 à 0,12 % en poids, de préférence de 0,05 à 0,10 % en poids, par rapport au poids total de la composition et calculé sous forme d'acide hyaluronique.

4. Composition selon l'une ou plusieurs des revendications précédentes, où (b) l'acide hyaluronique et/ou ses sels est/sont d'origine non animale et/ou où l'acide hyaluronique et/ou ses sels est/sont d'origine bactérienne, en particulier où l'acide hyaluronique et/ou ses sels est/sont de préférence produit(s) par fermentation de bactéries du type Streptococcus, en particulier Streptococcus lancefields, de préférence Streptococcus lancefields souche A, et/ou où la composition contient comme composant (b) au moins un hyaluronate de métal alcalin, de préférence l'hyaluronate de sodium.

5. Composition selon l'une ou plusieurs des revendications précédentes, contenant en outre (c) au moins un électrolyte, en particulier en des quantités de 0,005 % en poids à 1,5 % en poids, en particulier de 0,05 à 1,25 % en poids, de préférence de 0,25 à 1 % en poids, de préférence de 0,5 à 0,75 % en poids, par rapport au poids total de la composition, en particulier où ledit au moins un électrolyte est choisi parmi les sels organiques et/ou inorganiques de métal alcalin ou alcalino-terreux ainsi que leurs mélanges, de préférence parmi les sels inorganiques de métal alcalin ou alcalino-terreux, en particulier où le sel inorganique de métal alcalin ou alcalino-terreux est un chlorure, de préférence le chlorure de sodium.

6. Composition selon l'une ou plusieurs des revendications précédentes, contenant en outre (d) du sulfate de chondroïtine, en particulier en des quantités de 0,02 à 0,2 % en poids, de préférence de 0,03 à 0,16 % en poids, de préférence de 0,04 à 0,12 % en poids, de manière particulièrement préférée de 0,05 à 0,10 % en poids, par rapport au poids total de la composition, et/ou contenant en outre (e) du dexpanthénol et/ou ses dérivés, en particulier en des quantités de 0,0001 à 5 % en poids, de préférence de 0,001 à 4 % en poids, de préférence de 0,01 à 3 % en poids, de manière particulièrement préférée de 0,1 à 2 % en poids, par rapport au poids total de la composition et/ou contenant en outre (f) de l'ectoïne et/ou ses dérivés, en particulier en des quantités de 0,0001 à 5 % en poids, de préférence de 0,001 à 4 % en poids, de préférence de 0,01 à 3 % en poids, de manière particulièrement préférée de 0,1 à 2 % en poids, par rapport au poids total de la composition.

7. Composition selon l'une ou plusieurs des revendications précédentes, contenant en outre (g) au moins un anesthésique local, en particulier en des quantités de 0,0001 à 5 % en poids, de préférence de 0,001 à 4 % en poids, de préférence de 0,01 à 3 % en poids, de manière particulièrement préférée de 0,1 à 2 % en poids, par rapport au poids total de la composition, et/ou contenant en outre (h) des substances minérales et/ou des vitamines, en particulier des composés du zinc, de préférence des composés organiques du zinc, de préférence le gluconate de zinc, en particulier en des quantités de 0,0001 à 5 % en poids, de préférence de 0,001 à 4 % en poids, de préférence de 0,01 à 3 % en poids, de manière particulièrement préférée de 0,1 à 2 % en poids, par rapport au poids total de la composition.

8. Composition selon l'une ou plusieurs des revendications précédentes, où la composition se trouve sous forme liquide, en particulier sous forme d'une solution aqueuse ou d'une suspension aqueuse et/ou où la composition présente un pH de 5,5 à 7,5, en particulier de 6,0 à 7,0, de préférence de 6,2 à 6,8, et/ou où la composition présente, à une température de 20°C, une viscosité dynamique de 1000 à 12 000 mPa.s, en particulier de 2000 à 10 000 mPa.s, de préférence de 4000 à 9000 mPa.s, de préférence de 6000 à 8000 mPa.s.

9. Composition selon l'une ou plusieurs des revendications précédentes, en particulier sous forme d'une solution aqueuse ou d'une suspension aqueuse, contenant en combinaison
(a) de l'oxybutynine et/ou ses sels, de préférence le chlorhydrate d'oxybutynine, en particulier avec une teneur en substances actives de 0,01 à 0,2 mg/ml, en particulier de 0,01 à 0,15 mg/ml, de préférence de 0,02 à 0,1 mg/ml, de préférence de 0,03 à 0,08 mg/ml, de manière particulièrement préférée de 0,04 à 0,07 mg/ml, par rapport au volume de la composition et calculé sous forme d'oxybutynine,
(b) de l'acide hyaluronique et/ou ses sels, en particulier d'origine non animale, de préférence l'hyaluronate de sodium, de préférence l'hyaluronate de sodium d'origine non animale présentant une masse molaire moyenne (poids moléculaire) inférieure à 200 kDa, en particulier présentant une masse molaire dans la plage de 50 à 195 kDa, en particulier de 100 à 195 kDa, de préférence de 150 à 190 kDa, de préférence de 175 à 190 kDa, et/ou en particulier présentant une teneur en substance active de 0,2 à 2,0 mg/ml, en particulier de 0,3 à 1,6 mg/ml, de préférence de 0,4 à 1,2 mg/ml, de préférence de 0,5 à 1,0 mg/ml, de manière particulièrement préférée de 0,7 à 0,9 mg/ml, par rapport au volume de la composition et calculé sous forme d'acide hyaluronique, et
(c) le cas échéant au moins un électrolyte, de préférence le chlorure de sodium, en particulier en une teneur de 0,5 à 20 mg/ml, en particulier de 1 à 10 mg/ml, de préférence de 4 à 8 mg/ml,
en particulier où le rapport des quantités (rapport pondéral) de (a) oxybutynine et/ou ses sels, de préférence le chlorhydrate d'oxybutynine, d'une part à (b) acide hyaluronique et/ou ses sels, de préférence l'hyaluronate de sodium, d'autre part, dans la composition se situe dans la plage de 1:1 à 1:200, en particulier de 1:2 à 1:160, de préférence de 1:4 à 1:60, de préférence de 1:6 à 1:35, de manière particulièrement préférée de 1:10 à 1:20, de manière tout particulièrement préférée à environ 1:14, calculé sous forme d'oxybutynine à acide hyaluronique.

10. Composition selon l'une ou plusieurs des revendications précédentes destinée à l'instillation et/ou à l'application de préférence topique dans le domaine urogénital, en particulier dans la vessie.

11. Récipient contenant une composition selon l'une ou plusieurs des revendications précédentes, en particulier sous forme d'un flacon à perforer avec une fermeture de préférence stérile.

12. Dispositif d'application destiné à l'instillation et/ou à l'application de préférence topique dans le domaine urogénital, en particulier dans la vessie, contenant une composition selon l'une quelconque des revendications 1 à 10.

13. Utilisation d'une composition selon l'une quelconque des revendications 1 à 10 pour la préparation d'un médicament destiné au traitement prophylactique ou thérapeutique de maladies du tractus urogénital, en particulier de l'incontinence urinaire, de préférence d'une incontinence urinaire associée à une hyperactivité et/ou une hyperréflexie de l'appareil de détrusor (hyperactivité du détrusor et/ou hyperréflexie du détrusor), en particulier où la composition est utilisée pour la préparation d'un médicament destiné au traitement prophylactique ou thérapeutique d'une incontinence urinaire provoquée par une hyperactivité et/ou une hyperréflexie idiopathique et/ou neurogène du détrusor.

14. Utilisation selon la revendication 13 pour la préparation d'un médicament destiné au traitement prophylactique ou thérapeutique de l'incontinence motrice par impériosité et/ou en particulier de l'incontinence de réflexe spinale et/ou supraspinale.

15. Utilisation d'une composition selon l'une quelconque des revendications 1 à 10 pour la préparation d'un médicament destiné au traitement de maladies du tractus urogénital, en particulier de maladies inflammatoires de la vessie, de préférence de la cystite, de préférence de la cystite interstitielle, de la cystite radiogène et/ou de la cystite bactérienne chronique, de manière particulièrement préférée de la cystite interstitielle.
